# EUROPEAN PATENT APPLICATION

(11) **EP 3 889 574 A1**
(43) Date of publication of application: **06.10.2021**
(21) Application number: 19889957.7
(22) Date of filing: 29.01.2019
(51) Int. Cl.: G01N 3/34

(54) **FULL-ENVIRONMENT FULL-SCALE CYCLIC ACCELERATED LOADING TEST SYSTEM**

(30) Priority: 26.11.2018 CN 201811413312
(71) Applicant: Shandong Jiaotong University, Shandong 250023 (CN)
(72) Inventor: FENG, Jinxiang, Jinan, Shandong 250023 (CN); GUO, Xingyu, Jinan, Shandong 250023 (CN); ZHANG, Peng, Jinan, Shandong 250023 (CN); GUAN, Zhiguang, Jinan, Shandong 250023 (CN); ZHANG, Jiwei, Jinan, Shandong 250023 (CN); WU, Qingzhen, Jinan, Shandong 250023 (CN); JIA, Qian, Jinan, Shandong 250023 (CN); YANG, Fuguang, Jinan, Shandong 250023 (CN); RUAN, Jiuhong, Jinan, Shandong 250023 (CN); WANG, Huijun, Jinan, Shandong 250023 (CN); HAN, Ying, Jinan, Shandong 250023 (CN)
(74) Representative: Stiel, Jürgen
(86) International application number: PCT/CN2019/073718
(87) International publication number: WO 2020/107702

(57) **Abstract**

The present disclosure provides a total-environment full-scale cyclic accelerated loading experimental system, which pertains to a field of accelerated loading experimental systems. The total-environment full-scale cyclic accelerated loading experimental system includes a rack, and a power mechanism, a chain drive pair, a roller set, a guide rail, etc. installed on the rack. The power mechanism is connected to the roller set through the chain drive pair. The roller set is matched with an annular loading surface of the guide rail. In the present disclosure, in addition to an environmental unit being provided on the rack, revolutionary changes are made to structures of the guide rail, the roller set and the chain drive pair as well as connections thereof. The system of the present disclosure has a simple structure. The loading module, the environmental module and other modules are integrated. With a front traveling guide wheel and a rear traveling wheel, the system itself may perform a transition operation, which is convenient and achieves a high efficiency. In addition, no disassembly and assembly of the system is required during the transition, which saves labor and improves testing efficiency. With a technical solution of driving a plurality of sets of rolling wheels by using the chain drive pair, the experimental efficiency is greatly improved. Furthermore, the system has a simple structure and a high reliability, and needs low maintenance costs.

## Description

### TECHNICAL FIELD

The present disclosure relates to an accelerated loading experimental system, and in particular to a total-environment full-scale cyclic accelerated loading experimental system.

### BACKGROUND

Existing full-scale accelerated loading experimental systems mainly include following types:

ALF apparatus developed by Australia, which is one-way loaded and free in a return stroke, so that an experimental efficiency is low, and which does not have an environment simulation function.

HVS apparatus developed by America, which is one-way loaded and free in a return stroke, so that an experimental efficiency is low, and which does not have an environment simulation function.

MLS apparatus developed by South Africa, which is multi-wheel-group cyclically loaded with a high experimental efficiency, however, the total energy consumption is high, the failure rate is high, the maintenance is inconvenient and the reliability is low, the environment simulation function of which may only simulate high temperature conditions.

ALT apparatus developed by Shandong JiaoTong University in China, which is one-way loaded and free in a return stroke so that an experimental efficiency is low, an environmental chamber needed in simulating environment conditions has a large volume, so that a transition is difficult and the energy consumption is high.

In summary, in terms of the environmental simulation function, the MLS apparatus may only simulate high-temperature conditions by heating; the environmental chamber to be adjusted in the ALT apparatus has a large volume, so that the environmental simulation is difficult; and the existing full-scale accelerated loading apparatuses do not have a comprehensive total-environment simulation function such as high and low temperature, humidity, ultraviolet light emitting and rain; in terms of the experimental efficiency, except for the MLS apparatus of South Africa, the existing full-scale accelerated loading experimental systems are all one-way loaded and free in the return stroke, so that the experimental efficiency is low; the MLS apparatus adopts linear motor plate chain drive, in which a plurality of sets of steel guide wheels are outer side guided, which has a complex structure and produces high noise; the total energy consumption is high, the failure rate is high, the maintenance is inconvenient and the reliability is low.

### SUMMARY

Technical objective of the present disclosure is to provide a total-environment full-scale cyclic accelerated loading experimental system aiming at deficiencies of the related art, and is intended to solve the problem of low efficiency, complex structure, high energy consumption and difficult environmental simulation in the existing full-scale accelerated loading experimental system. Further, the present disclosure may significantly improve reliability.

To solve the technical problems, following technical solutions are adopted in the present disclosure.

A total-environment full-scale cyclic accelerated loading experimental system, including: a rack, and a power mechanism, a chain drive pair, a roller set, a guide rail, a control cabinet, a rear leg and a front leg installed on the rack, wherein the power mechanism is connected to the roller set through the chain drive pair, the roller set includes a loading wheel matched with an annular loading surface of the guide rail,
wherein an environmental unit is further provided on the rack;
wherein the guide rail is of a box structure filled with a shock-absorbing material, an upper loading surface of the guide rail is a flat surface, a lower loading surface of the guide rail is of a ship bottom shape and includes a straight section parallel to an experimental rolled road surface, and both ends of the upper loading surface and the lower loading surface are smoothly transitionally connected through a curved section;
wherein the system includes a plurality of roller sets, each roller set includes two loading wheels and more than one rolling wheel located between the two loading wheels and arranged coaxially with the two loading wheels, the two loading wheels move cyclically along the annular loading surface, and the rolling wheel moves synchronously to roll;
wherein the system includes two sets of chain drive pairs symmetrically arranged on both sides of the guide rail, each set of chain drive pair includes a driving sprocket, a driven sprocket, and a chain for linking the driving sprocket with the driven sprocket, two driving sprockets are arranged coaxially, two driven sprockets are arranged coaxially, axles of the two driving sprockets are connected to the power mechanism; and
wherein the chain is a closed annular structure formed by a plurality of chain segments connected sequentially, adjacent chain segments are connected by a coupling and a chain pin, both ends of a loading shaft of each roller set are connected to the chains of the two sets of chain drive pairs through the couplings.

Further, the environmental unit is a combination of more than one of an air conditioner, a heater, a humidifier, a spray device and an ultraviolet light emitting device.

Further, the plurality of roller sets are arranged at equal intervals along the chain, and at least two adjacent roller sets roll the road surface simultaneously.

Further, the system includes an even number of roller sets, two adjacent roller sets are arranged along the chain in parallel and tightly so as to form a group of roller sets, and at least one group of roller sets rolls the road surface.

Further, a clamping hole is provided at an end surface of the coupling, an end portion of the loading shaft of the roller set is mated and inserted into and fixedly connected to the clamping hole of the coupling, and a loading shaft axis of the roller set perpendicularly intersects with a longitudinal center line of the chain.

Further, a chain drive tensioner for adjusting spacing is further provided between the driving sprocket and the driven sprocket.

Further, a front traveling guide wheel and a rear traveling wheel are further respectively provided at a front side and a rear side of a bottom of the rack.

Further, the control cabinet includes a host, a display and a frequency converter.

Further, the power mechanism includes a motor and a decelerator connected to the motor.

Further, a generator set is further provided on the rack.

Compared with the related art, the total-environment full-scale cyclic accelerated loading experimental system of the present disclosure has following beneficial effects.

With a technical solution of driving a plurality of sets of rolling wheels by using the chain drive pair, the experimental efficiency is greatly improved. Furthermore, the system has a simple structure, a high reliability, and needs low maintenance costs.

The roller set and the chain are connected by the coupling, so that the problem of the chain driving a large load is solved, and the reliability of the apparatus is improved.

The environmental unit may achieve a total-environmental simulation of environmental conditions such as high temperature, low temperature, high humidity, rain, and ultraviolet light, so that the experimental function of the apparatus is improved. In addition, the environmental chamber has a small volume and has low energy consumption.

The chain drive tensioner is used to tension the chain so that the loading wheel always contacts with the guide rail, which avoids impact and vibration and improves the stability and reliability of the apparatus.

The system of the present disclosure has a simple structure. The loading module, the environmental module and other modules are integrated. With a front traveling guide wheel and a rear traveling wheel, the system itself may perform a transition operation, which is convenient and achieves a high efficiency. In addition, no disassembly and assembly of the system is required during the transition, which saves labor and improves the experimental efficiency.

### BRIEF DESCRIPTION OF THE DRAWINGS

FIG. 1 is a schematic diagram of a front view structure of the present disclosure;
FIG. 2 is a schematic diagram of an internal structure of FIG. 1;
FIG. 3 is a schematic diagram of a top view of an internal structure of the present disclosure.
FIG. 4 is a schematic diagram of a cross-sectional structure of a coupling.
FIG. 5 is a schematic structural diagram where a chain and a roller set are connected through a coupling.
FIG. 6 is a schematic diagram of a connection of a driving sprocket, a loading wheel and a rolling wheel of the present disclosure.
FIG. 7 is a schematic structural diagram of a guide rail of the present disclosure.
FIG. 8 is a schematic structural diagram of a roller set according to a second embodiment of the present disclosure.

### Reference numerals:

1. rack; 2. driving sprocket; 3. chain drive tensioner; 4. chain; 5. driven sprocket; 6. control cabinet; 7. rear leg; 8. real traveling wheel; 9. decelerator; 10. motor; 11. guide rail; 12. rolling wheel; 13. loading wheel; 14. front traveling guide wheel; 15. front leg; 16. environmental unit; 17. connector; 18. axle; 19. chain pin; 20. driving shaft; 21. driven shaft; 22. curve section; 23. upper loading surface; 24. lower loading surface; 25. generator set; 26. coupling; 27. clamping hole.

### DETAILED DESCRIPTION OF EMBODIMENTS

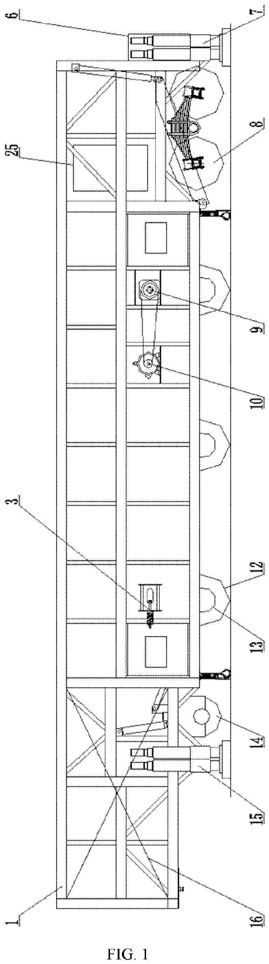

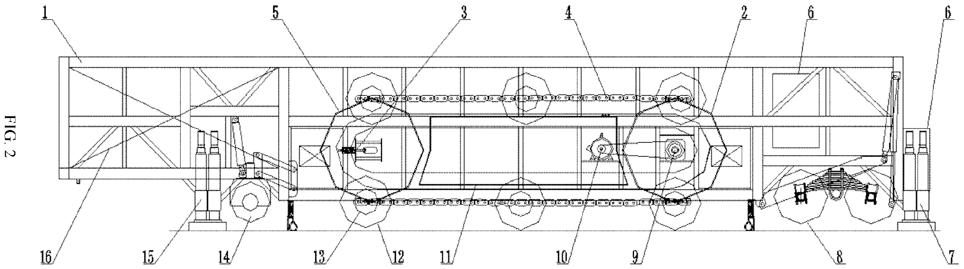

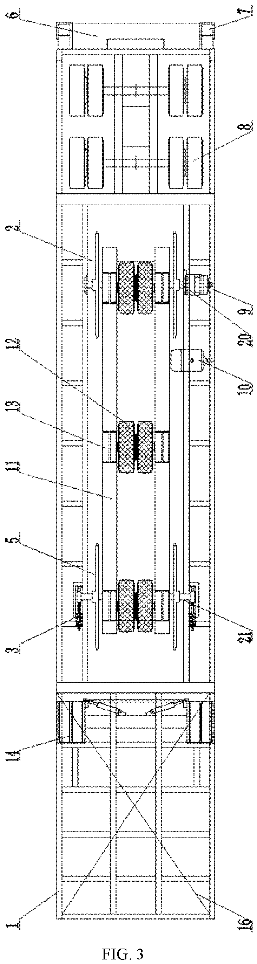

A total-environment full-scale cyclic accelerated loading experimental system of the present disclosure is described below in detail with reference to FIG. 1 to FIG. 8.

### First Embodiment

As shown in FIG. 1 to FIG. 3, the total-environment full-scale cyclic accelerated loading experimental system of the present disclosure includes a rack 1, and a power mechanism, a chain drive pair, a roller set, a guide rail 11, a control cabinet 6, an environmental unit 16, a rear leg 7 and a front leg 15 installed on the rack 1. The power mechanism includes a motor 10 and a decelerator 9 connected to the motor 10. The system includes two sets of chain drive pairs symmetrically arranged on both sides of the guide rail 11. Each set of chain drive pair includes a driving sprocket 2, a driven sprocket 5, and a chain 4 for linking the driving sprocket 2 with the driven sprocket 5. Two driving sprockets 2 are arranged coaxially, and two driven sprockets 5 are arranged coaxially. Axles of the two driving sprockets 2 are connected to the power mechanism.

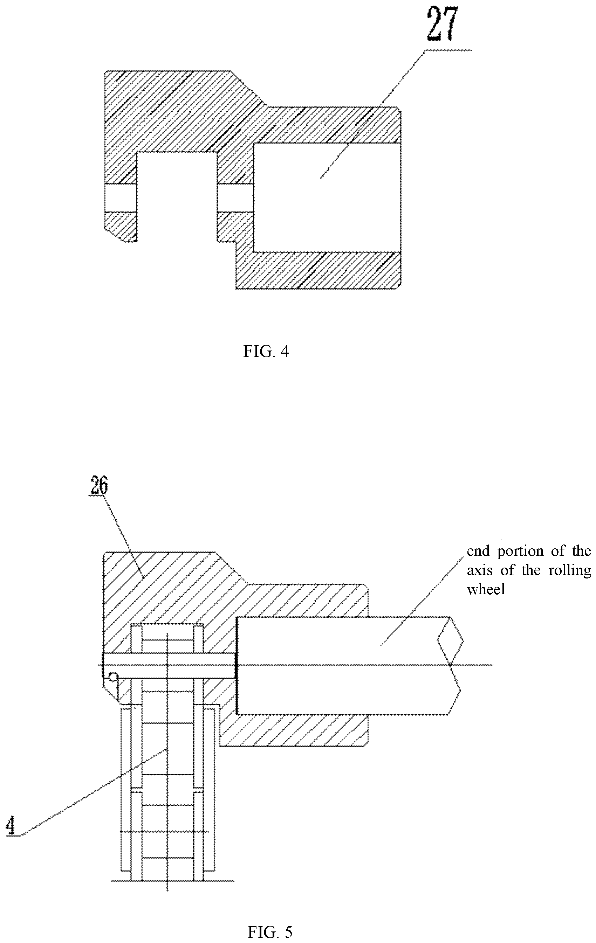

As shown in FIG. 4 to FIG. 6, the chain 4 is a closed annular structure formed by a plurality of chain segments connected sequentially. Adjacent chain segments are connected by a coupling 4 and a chain pin 19. Both ends of a loading shaft of each roller set are connected to the chains of the two sets of chain drive pairs through the couplings 26. The system includes a plurality of roller sets, each roller set includes two loading wheels 13 and more than one rolling wheels 12 located between the two loading wheels 13 and arranged coaxially with the two loading wheels 13. The two loading wheels 13 move cyclically along an annular loading surface, so that the rolling wheel 12 moves synchronously to roll. The plurality of roller sets are arranged at equal intervals along the chain, and at least two adjacent roller sets may roll the road surface simultaneously.

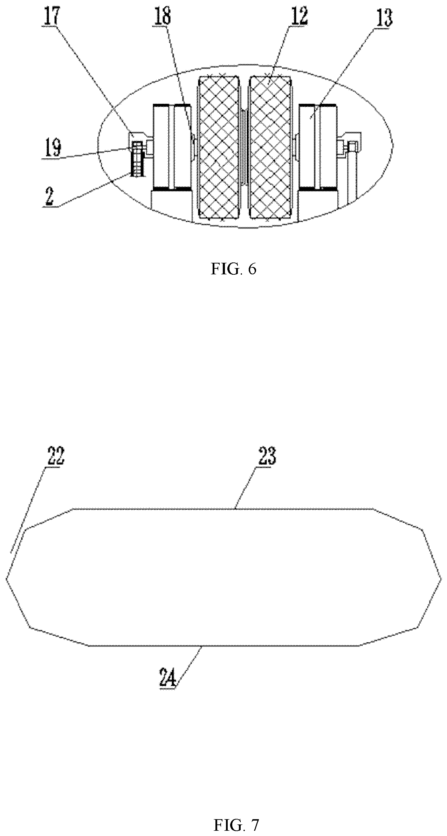

As shown in FIG. 7, the guide rail 11 is of a box structure filled with a shock-absorbing material. An upper loading surface 23 of the guide rail 11 is a flat surface, a lower loading surface 24 of the guide rail 11 is of a ship bottom shape, and the lower loading surface 24 includes a straight section parallel to an experimental rolled road surface. Both ends of the upper loading surface 23 and the lower loading surface 24 are smoothly transitionally connected through a curved section 22. The power mechanism is connected to the roller set through the chain drive pair. The loading wheel 13 of the roller set matched with the annular loading surface of the guide rail 11. The control cabinet 6 includes a host, a display and a frequency converter. The environmental unit includes an air conditioner, a heater, a humidifier, a spray device and an ultraviolet light emitting device.

A clamping hole 27 is provided at an end surface of the coupling 26. An end portion of the loading shaft of the roller set is mated and inserted into and fixedly connected to the clamping hole 27 of the coupling 26. A loading shaft axis of the roller set perpendicularly intersects with a longitudinal center line of the chain.

A chain drive tensioner 3 for adjusting spacing is further provided between the driving sprocket 2 and the driven sprocket 5.

A front traveling guide wheel 14 and a rear traveling wheel 8 are further respectively provided at a front side and a rear side of a bottom of the rack 1.

### Second Embodiment

As shown in FIG. 1 to FIG. 3, a total-environment full-scale cyclic accelerated loading experimental system of the present disclosure includes a rack 1, and a power mechanism, a chain drive pair, a roller set, a guide rail 11, a control cabinet 6, an environmental unit 16, a generator set, a rear leg 7 and a front leg 15 installed on the rack 1. The power mechanism includes a motor 10 and a decelerator 9 connected to the motor 10. The system includes two sets of chain drive pairs symmetrically arranged on both sides of the guide rail 11. Each set of chain drive pair includes a driving sprocket 2, a driven sprocket 5, and a chain 4 for linking the driving sprocket 2 with the driven sprocket 5. Two driving sprockets 2 are arranged coaxially, and two driven sprockets 5 are arranged coaxially. Axles of the two driving sprockets 2 are connected to the power mechanism.

As shown in FIG. 4 to FIG. 6, the chain 4 is a closed annular structure formed by a plurality of chain segments connected sequentially. Adjacent chain segments are connected by a coupling 26 and a chain pin 19. Both ends of a loading shaft of each roller set are connected to the chains of the two sets of chain drive pairs through the couplings 26. The system includes a plurality of roller sets, each roller set includes two loading wheels 13 and more than one rolling wheel 12 located between the two loading wheels 13 and arranged coaxially with the two loading wheels 13. The two loading wheels 13 move cyclically along the annular loading surface, so that the rolling wheel 12 moves synchronously to roll.

As shown in FIG. 7, the guide rail 11 is of a box structure filled with a shock-absorbing material. An upper loading surface 23 of the guide rail 11 is a flat surface, a lower loading surface 24 of the guide rail 11 is of a ship bottom shape and includes a straight section parallel to an experimental rolled road surface. Both ends of the upper loading surface 23 and the lower loading surface 24 are smoothly transitionally connected through a curved section 22. The power mechanism is connected to the roller set through the chain drive pair. The loading wheel 13 of the roller set matched with the annular loading surface of the guide rail 11. The control cabinet 6 includes a host, a display and a frequency converter. The environmental unit includes an air conditioner, a heater, a humidifier, a spray device and an ultraviolet light emitting device.

A clamping hole 27 is provided at an end surface of the coupling 26. An end portion of the loading shaft of the roller set is mated and inserted into and fixedly connected to the clamping hole 27 of the coupling 26. A loading shaft axis of the roller set perpendicularly intersects with a longitudinal center line of the chain.

A chain drive tensioner 3 for adjusting spacing is further provided between the driving sprocket 2 and the driven sprocket 5.

A front traveling guide wheel 14 and a rear traveling wheel 8 are further respectively provided at a front side and a rear side of a bottom of the rack 1.

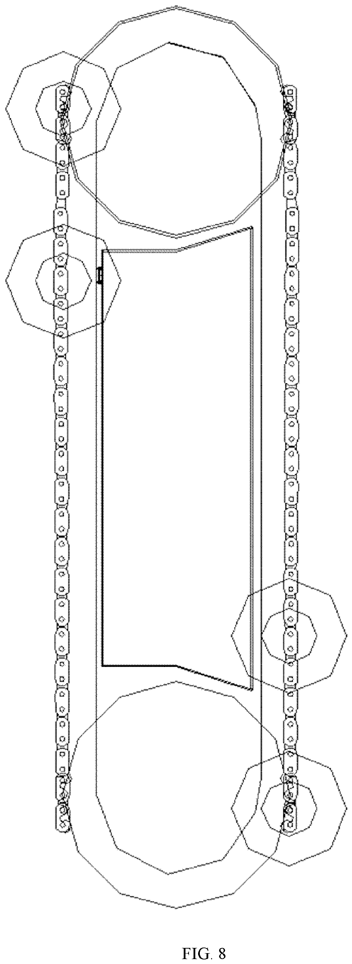

As shown in FIG. 8, the system includes an even number of roller sets. Two adjacent roller sets are arranged along the chain in parallel and tightly so as to form a group of roller sets. At least one group of roller sets rolls the road surface.

## Claims

1. A total-environment full-scale cyclic accelerated loading experimental system, comprising: a rack (1), and a power mechanism, a chain drive pair, a roller set, a guide rail (11), a control cabinet (6), a rear leg (7) and a front leg (15) installed on the rack (1), wherein the power mechanism is connected to the roller set through the chain drive pair, the roller set comprises a loading wheel (13) matched with an annular loading surface of the guide rail (11),
wherein an environmental unit (16) is further provided on the rack (1);
wherein the guide rail (11) is of a box structure filled with a shock-absorbing material, an upper loading surface (23) of the guide rail (11) is a flat surface, a lower loading surface (24) of the guide rail (11) is of a ship bottom shape and comprises a straight section parallel to an experimental rolled road surface, and both ends of the upper loading surface (23) and the lower loading surface (24) are smoothly transitionally connected through a curved section (22);
wherein the system comprises a plurality of roller sets, each roller set comprises two loading wheels (13) and more than one rolling wheel (12) located between the two loading wheels (13) and arranged coaxially with the two loading wheels, the two loading wheels (13) move cyclically along the annular loading surface, and the rolling wheel (12) moves synchronously to roll;
wherein the system comprises two sets of chain drive pairs symmetrically arranged on both sides of the guide rail (11), each set of chain drive pair comprises a driving sprocket (2), a driven sprocket (5), and a chain (4) for linking the driving sprocket (2) with the driven sprocket (5), two driving sprockets (2) are arranged coaxially, two driven sprockets (5) are arranged coaxially, axles of the two driving sprockets (2) are connected to the power mechanism; and
wherein the chain (4) is a closed annular structure formed by a plurality of chain segments connected sequentially, adjacent chain segments are connected by a coupling (26) and a chain pin (19), both ends of a loading shaft of each roller set are connected to the chains of the two sets of chain drive pairs through the couplings (26).

2. The total-environment full-scale cyclic accelerated loading experimental system of claim 1, wherein the environmental unit is a combination of more than one of an air conditioner, a heater, a humidifier, a spray device and an ultraviolet light emitting device.

3. The total-environment full-scale cyclic accelerated loading experimental system of claim 1 or 2, wherein the plurality of roller sets are arranged at equal intervals along the chain, and at least two adjacent roller sets roll the road surface simultaneously.

4. The total-environment full-scale cyclic accelerated loading experimental system of claim 1 or 2, wherein the system comprises an even number of roller sets, two adjacent roller sets are arranged along the chain in parallel and tightly so as to form a group of roller sets, and at least one group of roller sets rolls the road surface.

5. The total-environment full-scale cyclic accelerated loading experimental system of claim 1 or 2, wherein a clamping hole (27) is provided at an end surface of the coupling (26), an end portion of the loading shaft of the roller set is mated and inserted into and fixedly connected to the clamping hole (27) of the coupling (26), and a loading shaft axis of the roller set perpendicularly intersects with a longitudinal center line of the chain.

6. The total-environment full-scale cyclic accelerated loading experimental system of claim 1 or 2, wherein a chain drive tensioner (3) for adjusting spacing is further provided between the driving sprocket (2) and the driven sprocket (5).

7. The total-environment full-scale cyclic accelerated loading experimental system of claim 1 or 2, wherein a front traveling guide wheel (14) and a rear traveling wheel (8) are further respectively provided at a front side and a rear side of a bottom of the rack (1).

8. The total-environment full-scale cyclic accelerated loading experimental system of claim 1 or 2, wherein the control cabinet (6) comprises a host, a display and a frequency converter.

9. The total-environment full-scale cyclic accelerated loading experimental system of claim 1 or 2, wherein the power mechanism comprises a motor (10) and a decelerator (9) connected to the motor (10).

10. The total-environment full-scale cyclic accelerated loading experimental system of claim 1 or 2, wherein a generator set (25) is further provided on the rack (1).
